(19) Europäisches Patentamt
European Patent Office
Office européen des brevets

(11) EP 2 683 441 B1

(12) **EUROPEAN PATENT SPECIFICATION**

(45) Date of publication and mention of the grant of the patent:
**28.09.2016 Bulletin 2016/39**

(21) Application number: **12709829.1**

(22) Date of filing: **09.03.2012**

(51) Int Cl.:
***A61N 5/06*** *(2006.01)*

(86) International application number:
**PCT/EP2012/054060**

(87) International publication number:
**WO 2012/123343 (20.09.2012 Gazette 2012/38)**

(54) **FOCAL PHOTODYNAMIC THERAPY PLANNING METHODS**

FOKALE FOTODYNAMISCHE THERAPIE PLANUNGSVERFAHREN

PROCÉDÉS DE PLANIFICATION DE THÉRAPIE PHOTODYNAMIQUE FOCALISÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.03.2011 US 201161451939 P**

(43) Date of publication of application:
**15.01.2014 Bulletin 2014/03**

(73) Proprietor: **Steba Maor SA**
**2613 Luxembourg (LU)**

(72) Inventors:
- **ABENHAIM, Lucien**
  **London**
  **Bedfordshire EC1 R5 (GB)**
- **CHARBIT, Suzy**
  **F-94000 Creteil (FR)**
- **GAILLAC, Bertrand**
  **F-75010 Paris (FR)**

(74) Representative: **L'Helgoualch, Jean et al**
**Ipsilon**
**Le Centralis**
**63, avenue du Général Leclerc**
**92340 Bourg-la-Reine (FR)**

(56) References cited:
**US-A1- 2011 034 971**

- **SEAN R H DAVIDSON ET AL: "Treatment planning and dose analysis for interstitial photodynamic therapy of prostate cancer; Treatment planning and analysis for prostate PDT", PHYSICS IN MEDICINE AND BIOLOGY, TAYLOR AND FRANCIS LTD. LONDON, GB, vol. 54, no. 8, 21 April 2009 (2009-04-21) , pages 2293-2313, XP020149927, ISSN: 0031-9155, DOI: 10.1088/0031-9155/54/8/003**

EP 2 683 441 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### 1. BACKGROUND

Some men with early prostate cancer seek an alternative to whole gland therapy, on the one hand, and active surveillance without therapeutic intervention, on the other. Selective therapies that treat the cancer while preserving normal prostate tissue are increasingly sought. Such selective therapies include tumor-targeted approaches capable of discriminating neoplastic from benign tissue, and focal therapies, in which selectivity is achieved by spatially-directed focal ablation.

Among focal therapies, photodynamic therapy (PDT) - in which a photosensitizing agent is administered systemically and is photoactivated locally to the tumor site - has become an increasingly attractive option given the development of a new generation of photosensitizing agents with improved properties. Notable among these new agents are metal-containing derivatives of bacteriochlorophylls, two of which have been advanced into human clinical trials in the past decade, Pd-Bacteriopheophorbide (padoporfin; WST09; TOOKAD®)- *see* Koudinova et al., "Photodynamic therapy with Pd-Bacteriopheophorbide (TOOKAD): successful in vivo treatment of human prostatic small cell carcinoma xenografts," Int. J. Cancer 104(6):782-9 (2003); Weersink et al., "Techniques for delivery and monitoring of TOOKAD (WST09)-mediated photodynamic therapy of the prostate: clinical experience and practicalities," J Photochem Photobiol B. 79(3):211-22 (2005); Trachtenberg et al., "Vascular targeted photodynamic therapy with palladium-bacteriopheophorbide photosensitizer for recurrent prostate cancer following definitive radiation therapy: assessment of safety and treatment response," J Urol. 178(5): 1974-9 (2007); Trachtenberg et al., "Vascular-targeted photodynamic therapy (padoporfin, WST09) for recurrent prostate cancer after failure of external beam radiotherapy: a study of escalating light doses," BJU Int. 102(5):556-62 (2008); Madar-Balakirski et al., "Permanent occlusion of feeding arteries and draining veins in solid mouse tumors by vascular targeted photodynamic therapy (VTP) with Tookad," PLoS Once 5(4):e10282 (2010) - and more recently, an improved anionic derivative thereof, Palladium $3^1$-oxo-15-methoxycarbonylmethyl-rhodobacteriochlorin $13^1$-(2-sulfoethyl) amide (WST11; STAKEL®; TOOKAD® Soluble™), *see* Mazor et al., "WST-11, A Novel Water-soluble Bacteriochlorophyll Derivative: Cellular Uptake, Pharmacokinetics, Biodistribution and Vascular-targeted Photodynamic Activity Using melanoma Tumors as a Model," Photochemistry & Photobiology 81:342-351 (2005); Brandis et al., "Novel Water-soluble Bacteriochlorophyll Derivatives for Vascular-targeted Photodynamic Therapy: Synthesis, solubility, Phototoxicity and the Effect of Serum Proteins," Photochemistry & Photobiology 81:983-993 (2005); Ashur et al., "Photocatalytic Generation of Oxygen Radicals by the Water-Soluble Bacteriochlorophyll Derivative WST-11, Noncovalently Bound to Serum Albumin," J. Phys. Chem. A 113:8027-8037 (2009).

One of the challenges facing photodynamic therapy is the need to define a prospective treatment plan that will direct the placement of optic fibers so as to deliver a treatment-effective light dose in the desired three dimensional volume of prostate tissue, without causing unacceptable collateral damage to other structures, such as the urethra and rectum. The complex interaction among light, photosensitizer, and oxygen, as well as the heterogeneity in light and drug distribution in the prostate, make current approaches to treatment planning computationally Planning methods for interstitial photodynamic therapy using optical fibers are disclosed by Davidson et al., "Treatment planning and dose analysis for interstitial photodynamic therapy of prostate cancer," Phys. Med. Biol. 54:2293-2313 (2009). The computational requirements preclude real-time intra-operative adjustment.

A second challenge arises from the clinical observation that there exists a treatment-effective threshold light dose, and that this threshold dose appears to vary widely among patients who receive the same photosensitizer and in whom fiber placement and light dose were planned according to the same algorithm. Davidson et al., "Treatment planning and dose analysis for interstitial photodynamic therapy of prostate cancer," Phys. Med. Biol. 54:2293-2313 (2009). This poses a challenge in selecting light dosages in advance of treatment, and thus in planning effective therapy.

There exists, therefore, a continuing need for methods by which the treatment-effective threshold dose can be defined in advance of treatment. There exists a further need for treatment planning software that incorporates such treatment-effective threshold dose calculation into the planning algorithm.

### 2. SUMMARY

Using the photosensitizing agent, Palladium $3^1$-oxo-15-methoxycarbonylmethyl-rhodobacteriochlorin $13^1$-(2-sulfoethyl) amide (WST11; TOOKAD® Soluble™), we have discovered that a Light Density Index ("LDI") can be calculated that predicts the efficacy of photodynamic therapy of prostate tumors. The prediction of efficacy has been validated by MRI as early as 1 week post-treatment, and further confirmed by negative biopsy at 6 months post-treatment. The LDI can be used to define a treatment-effective threshold light dose from historical data, providing a readily-calculable dose parameter that can be used in prospective treatment planning to increase likelihood of successful treatment, without adding significantly, and potentially reducing, computational complexity while increasing likelihood of therapeutic success.

Accordingly, in a first aspect, a method of treating prostate cancer is disclosed. The method comprises systemically administering a photosensitizing agent to a patient having a prostate tumor, and then activating the photosensitizing agent by delivering light of appropriate wavelength through at least one optical fiber positioned proximal to the tumor, wherein the administered light dose is at or above a prior-determined treatment-effective light density index (LDI) threshold.

In various embodiments, the treatment-effective LDI threshold is prior-determined from historical data obtained using the same photosensitizing agent. In certain embodiments, the historical data are obtained using the same photosensitizing agent, administered at the same systemic dosage, at times from historical data obtained using the same photosensitizing agent, administered at the same systemic dosage, and same wavelength of delivered light.

In various typical embodiments, the photosensitizing agent is administered intravenously.

In certain embodiments, the photosensitizing agent is Palladium $3^1$-oxo-15-methoxycarbonylmethyl-rhodobacteriochlorin $13^1$-(2-sulfoethyl) amide, or pharmaceutically acceptable salts thereof, including the dipotassium salt. Palladium $3^1$-oxo-15-methoxycarbonylmethyl-rhodobacteriochlorin $13^1$-(2-sulfoethyl) amide, or pharmaceutically acceptable salts thereof, is in certain embodiments administered intravenously at 3 - 6 mg/kg, including at a dose of 4 mg/kg. In certain embodiments using this photosensitizing agent, the dose of light delivered is 200 J/cm and the LDI threshold is 1.0.

In some embodiments, the activating light is delivered through a plurality of optical fibers, typically positioned using a perineal brachytherapy template. Typically, the light is delivered at a wavelength that approximates an absorption maximum of the systemically administered photosensitizing agent.

In a related aspect, an improvement is presented to methods of photodynamic treatment of prostate cancer in which a photosensitizing agent is administered systemically and then activated by delivery of light of appropriate wavelength through at least one optical fiber positioned proximal to the tumor. The improvement comprises delivering a light dose at or above a prior-determined treatment-effective light density index (LDI) threshold.

In a further aspect, the treatment-effective light density index threshold is used in improved methods of planning patient-specific photodynamic treatment of prostate cancer, including planning of vascular-targeted photodynamic treatment of prostate cancer. The improvement comprises setting the total length of illuminating fiber to be used for treatment based upon the planned treatment volume (PTV) and a prior-determined treatment-effective light density index threshold.

In typical embodiments, the total length of illuminating fiber is calculated as the product of PTV and a prior-determined treatment-effective light density index threshold, or scalar multiple thereof. The treatment-effective LDI threshold is typically prior-determined from historical data from use of the same photosensitizing agent, often from historical data in which the same photosensitizing agent, administered at the same systemic dosage, was used. In certain embodiments, the treatment-effective LDI threshold is determined from historical data from use of the same photosensitizing agent, administered at the same systemic dosage, same wavelength of delivered light, and same light density.

In a further aspect, a computer program product for treatment planning is presented. The program product comprises a computer usable medium having computer readable program code embodied therein, the computer readable program code adapted to be executed by a computer to implement a method for producing an improved patient-specific treatment plan for photodynamic therapy of prostate cancer. The computer-executed method comprises the step of setting the total length of illuminating fibers needed for effective therapy based upon the planned treatment volume (PTV) and a prior-determined treatment-effective light density index threshold.

In some embodiments, the total length of illuminating fibers is calculated as the product of PTV and prior-determined treatment-effective light density index threshold, or scalar multiple thereof. The treatment-effective LDI threshold is, in some embodiments, prior-determined from historical data in which the same photosensitizing agent was used as that intended for the use being planned. In various embodiments, the treatment-effective LDI threshold is prior-determined from historical data obtained from use of the same photosensitizing agent, administered at the same systemic dosage, and from the same photosensitizing agent, administered at the same systemic dosage, same wavelength of delivered light, and same light density.

In a further aspect, an assistance method, implemented by computer, for the planning of treatment of a patient by photodynamic therapy is presented, in which a predefined photosensitizing agent must be administered to the patient, and then subjected to illumination at a predetermined wavelength through at least one illuminating fiber designed to be introduced over a length of insertion into the treatment area. The assistance method comprises calculating the planned treatment volume, PTV, of the treatment area; determining a treatment-effective light density index, LDI, threshold; and setting then the total length of said at least one illuminating fiber to be used for treatment based upon the planned treatment volume, PTV, and said prior-determined treatment-effective light density index threshold

The invention is as defined in the appended claims.

## 3. DETAILED DESCRIPTION

Using the photosensitizing agent, Palladium $3^1$-oxo-15-methoxycarbonylmethyl-rhodobacteriochlorin $13^1$-(2-sulfoethyl) amide (WST11; TOOKAD® Soluble™), we have discovered that a Light Density Index ("LDI") can be calculated

that predicts the efficacy of photodynamic therapy of prostate tumors. The prediction of efficacy has been validated by MRI as early as 1 week post-treatment, and further confirmed by negative biopsy at 6 months post-treatment. The LDI can be used to define a treatment-effective threshold light dose from historical data, providing a readily-calculable dose parameter that can be used in prospective treatment planning to increase likelihood of successful treatment, without adding significantly, and potentially reducing, computational complexity, while concomitantly increasing likelihood of therapeutic success.

Accordingly, in a first aspect, a method of treating prostate cancer is provided. The method comprises systemically administering a photosensitizing agent to a patient having a prostate tumor, and then activating the photosensitizing agent by delivering light of appropriate wavelength through at least one optical fiber positioned proximal to the tumor, wherein the administered light dose is at or above a prior-determined treatment-effective light density index (LDI) threshold.

LDI

The Light Density Index ("LDI") is calculated as

$$LDI = \Sigma(n)L/PTV,$$

where $\sum(n)L$ is the total length of all illuminating fibers and PTV is the planned treatment volume. In typical embodiments, the length of all illuminating fibers is measured in centimeters, and the planned treatment volume is measured in milliliters.

The patient-specific PTV used in calculating the LDI is planned using known treatment planning approaches. In some embodiments, the PTV is derived by volume reconstruction from a series of MRI images of the patient's prostate, typically a transverse series, on a plurality of which, typically on all of which, the tumor margin has been outlined. The outline of the tumor margin on sectional images is typically performed by a radiologist or surgeon, although in certain embodiments discrimination of the tumor margin is performed by image recognition software, which is typically thereafter reviewed by a radiologist or surgeon. Volume reconstruction is performed using standard digital image processing techniques and algorithms. In typical embodiments, the PTV is planned to include an additional enveloping volume to ensure that treatment is sufficient to fully include the actual tumor margin. In such embodiments, the treatment planning software typically adds the user-chosen or software-predetermined margin to each two-dimensional sectional image in which the tumor margin has been circumscribed. In some embodiments, the margin can be added after the volume reconstruction, although this computationally more complex approach is presently not preferred. In some embodiments, the PTV is calculated according to the methods described in Davidson et al., "Treatment planning and dose analysis for interstitial photodynamic therapy of prostate cancer," Phys. Med. Biol. 54:2293-2313 (2009).

Treatment-effective LDI threshold

The treatment-effective LDI threshold is determined prior to treatment. In typical embodiments, the treatment-effective LDI threshold is prior-determined from historical clinical data.

In typical embodiments, the treatment-effective LDI threshold is determined by first correlating the magnitude of the treatment LDI for each of a series of historical patients with one or more later-observed patient-specific outcomes. The later-observed outcomes are chosen from art-accepted outcomes, including clinical outcomes, such as post-treatment survival, change in tumor stage or grade, or more typically, from radiologic and/or pathologic outcomes that are art-recognized as useful surrogates, such as evidence of tissue necrosis on post-treatment MRI, or percentage of negative biopsies post-treatment.

In some embodiments, the treatment LDI is calculated from historical data using the actual treated volume (ATV) instead of the historical prospective PTV. The ATV is usefully calculated from the area of necrosis observed on post-treatment MRI images, such as MRI images taken at 1 week post-treatment, 1 month post-treatment, 2 months post-treatment, 3 months post-treatment, and/or 6 months post-treatment. In certain embodiments, the ATV is derived by volume reconstruction from a series of post-treatment MRI images of the patient's prostate, typically a transverse series, on a plurality of which, typically on all of which, the margins of the necrotic area, or hypoperfused area, has been outlined. The outline of the necrotic or hypoperfused area on sectional images is typically performed by a radiologist or surgeon, although in certain embodiments, discrimination of the margin is performed by image recognition software, which is typically thereafter reviewed by a radiologist or surgeon. Volume reconstruction is performed using standard digital image processing techniques and algorithms.

In typical embodiments, standard statistical approaches will be applied to the correlated treatment LDI and outcome data to determine a treatment-effective threshold that provides a desired degree of statistical confidence. For

example, in Example I, below, we identify a treatment-effective LDI threshold having a P value of < 0.01 with respect to predicting necrosis volume on MRI at 1 week post-treatment as a percentage of the PTV, and also having a P value < 0.01 with respect to the percentage of negative biopsies at 6 months post-treatment. Any chosen treatment-effective LDI threshold may provide different magnitudes of statistical significance with respect to different outcomes.

Because the treatment-effective LDI threshold may differ depending on the choice of photosensitizing agent, its systemic dosage, and the irradiating wavelength delivered locally to the prostate, the treatment-effective LDI threshold is usefully derived from historical data drawn from prior clinical use of the same photosensitizing agent to be used in the subject patient. In some embodiments, the historical data are from use of the same photosensitizing agent, administered at the same systemic dosage, to be used in the subject patient. In some embodiments, the historical data are from use of the same photosensitizing agent, administered at the same systemic dosage, and irradiated with the same wavelength to be used in the subject patient. In addition, because the treatment-effective LDI threshold may differ depending on the light density (*e.g.*, in Joules/cm) delivered through each fiber, the treatment-effective LDI threshold is usefully derived from historical data drawn from prior clinical use of the same light density to be used in the subject patient.

The prior-determined treatment-effective LDI threshold does not require recalculation from historical data for each patient to be treated. In typical embodiments, the treatment-effective LDI threshold will be treated as a constant, typically user-entered, by treatment planning algorithms. However, it is contemplated that the treatment-effective LDI threshold will be recalculated on a periodic basis as additional historical data become available, such as the data on additional patients, and/or additional outcome data on patients included in the prior calculation. Furthermore, in certain embodiments, the treatment-effective LDI threshold will be calculated separately for defined subpopulations of historical patients, and the treatment-effective LDI threshold used for administering treatment to a given patient will be chosen based on the patient's similarity to the historical subpopulation.

Photosensitizing agents

The efficacy of the LDI parameter to predict treatment efficacy was demonstrated using the photosensitizing agent, Palladium $3^1$-oxo-15-methoxycarbonylmethyl-rhodobacteriochlorin $13^1$-(2-sulfoethyl) amide (WST11; TOOKAD® Soluble™) as the dipotassium salt.

Thus, in a preferred embodiment of the methods of this aspect of the present invention, the photosensitizing agent is Palladium $3^1$-oxo-15-methoxycarbonylmethyl-rhodobacteriochlorin $13^1$-(2-sulfoethyl) amide, or a pharmaceutically acceptable salt thereof. The WST11 compound in its un-ionized form has the structure given below, in formula (Ia), in which the tetrapyrrole carbons are numbered according to standard IUPAC nomenclature:

(Ia)

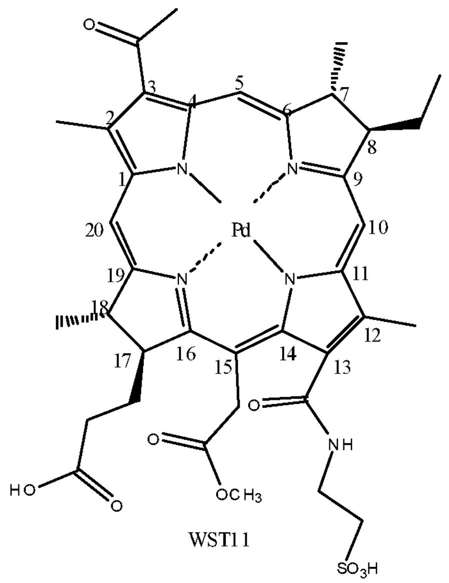

WST11

In various embodiments, pharmaceutically acceptable WST11 salts usefully include a counterion selected from monovalent and divalent alkaline and alkaline earth metal cations, such as one or more of $K^+$, $Na^+$, $Li^+$, and $Ca^{2+}$. In an embodiment that is at present particularly preferred, the dipotassium salt is used, as shown in Formula Ib:

(Ib)

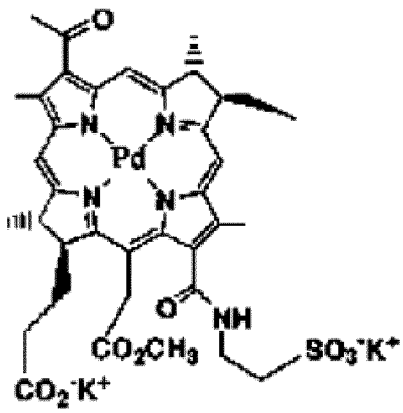

The compounds of formulae Ia and Ib are prepared according to known procedures. See WO 2004/045492 and US pre-grant application publication no. US 2006/01422260 A1, the disclosures of which are incorporated herein by reference in their entireties.

In other embodiments, the photosensitizing agent is a compound of Formula II:

(II)

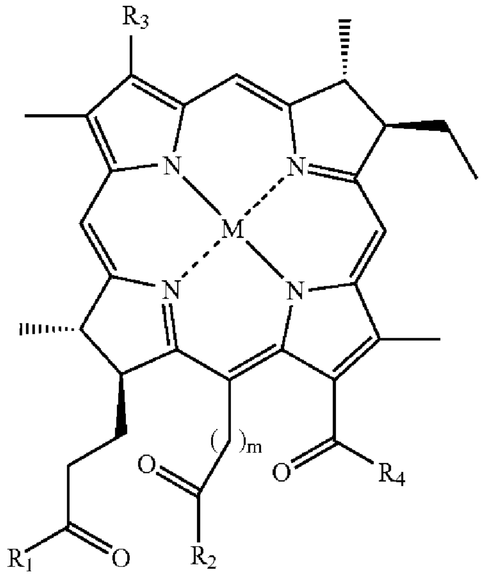

wherein

M represents 2H or a metal atom selected from divalent Pd, Pt, Co, Sn, Ni, Cu, Zn and Mn, and trivalent Fe, Mn and Cr;
$R_1$, $R_2$, and $R_4$ each independently is Y-$R_5$;
Y is O, S or $NR_5R_6$;
$R_3$ is selected from $-CH=CH_2$, $-C(=O)-CH_3$, $-C(=O)-H$, $-CH=NR_7$, $-C(CH_3)=NR_7$, $-CH_2-OR_7$, $-CH_2-SR_7$, $-CH_2-NR_7R'_7$, $-CH(CH_3)-OR_7$, $-CH(CH_3)-SR_7$, $-CH(CH_3)-NR_7R'_7$, $-CH(CH_3)Hal$, $-CH_2-Hal$, $-CH_2-R_7$, $-CH=CR_7R'_7$, $-C(CH_3)=CR_7R'_7$, $-CH=CR_7Hal$, $-C(CH_3)=CR_7Hal$, and $-C\equiv CR_7$;
$R_5$, $R_6$, $R_7$ and $R'_7$ each independently is H or is selected from the group consisting of:

(a) $C_1$-$C_{25}$ hydrocarbyl optionally containing one or more heteroatoms, carbocyclic or heterocyclic moieties, and/or optionally substituted by one or more functional groups selected from the group consisting of halogen, oxo, OH, SH, CHO, $NH_2$, $CONH_2$, a negatively charged group, and an acidic group that is converted to a negatively charged group at the physiological pH;
(b) a residue of an amino acid, a peptide or of a protein; and
(c) when Y is O or S, $R_5$ may further be $R_8^+$;

m is 0 or 1; and
$R_8^+$ is $H^+$ or a cation;

provided that:

(i) at least one, preferably two, of $R_5$, $R_6$, $R_7$ and $R'_7$ is a hydrocarbon chain as defined in (a) above substituted by a negatively charged group or by an acidic group that is converted to a negatively charged group at the physiological pH; or
(ii) at least one, preferably two, of $R_1$, $R_2$, and $R_4$ is OH, SH, $O^-R_8^+$ or $S^-R_8^+$; or
(iii) at least one of $R_1$, $R_2$, and $R_4$ is OH, SH, $O^-R_8^+$ or $S^-R_8^+$ and at least one of $R_5$, $R_6$, $R_7$ and $R'_7$ is a hydrocarbon chain substituted by a negatively charged group or by an acidic group that is converted to a negatively charged group at the physiological pH; or
(iv) at least one of $R_1$, $R_2$, and $R_4$ is OH, SH, $O^-R_8^+$ or $SR_8^+$ and at least one of $R_5$, $R_6$, $R_7$ and $R'_7$ is a residue of an amino acid, a peptide or of a protein; or
(v) at least one of $R_5$, $R_6$, $R_7$ and $R'_7$ is a hydrocarbon chain substituted by a negatively charged group or by an acidic group that is converted to a negatively charged group at the physiological pH and at least one of $R_5$, $R_6$, $R_7$ and $R'_7$ is a residue of an amino acid, a peptide or of a protein;

but excluding the compounds of formula I wherein M is as defined, $R_3$ is $-C(=O)CH_3$, $R_1$ is OH or $OR_8^+$ and $R_2$ is $-OCH_3$, and the compound of formula II wherein M is 2H, $R_3$ is $-C(=O)CH_3$, $R_1$, $R_2$ and $R_4$ are OH, and m is 0 or 1.

In various embodiments of photosensitizing agents of formula II, the negatively charged groups are selected from the group consisting of $COO^-$, $COS^-$, $SO_3^-$, and/or $PO_3^{2-}$. In various embodiments, the acidic groups that are converted to negatively charged groups at physiological pH are selected from the group consisting of COOH, COSH, $SO_3H$, and/or $PO_3H_2$. In certain embodiments, $R_1$ is $Y-R_5$; Y is O, S or NH; and $R_5$ is a hydrocarbon chain substituted by functional groups selected from OH, SH, $SO_3H$, $NH_2$, $CONH_2$, COOH, COSH, $PO_3H_2$. In selected embodiments, $R_5$ is the residue of an amino acid, a peptide or a protein. Usefully, M is a divalent palladium atom.

In certain embodiments of photosensitizing agents of formula II,

M represents 2H, divalent Pd, Cu, or Zn or trivalent Mn;
$R_1$ is $-O^-R_8^+$, $-NH-(CH_2)_n-SO_3^-R_8^+$, $-NH-(CH_2)_n-COO^-R_8^+$;$-NH-(CH_2)_n-PO_3^{2-}(R_8^+)_2$; or $Y-R_5$ wherein Y is O, S or NH and $R_5$ is the residue of an amino acid, a peptide or a protein;
$R_2$ is $C_1$-$C_6$ alkoxy such as methoxy, ethoxy, propoxy, butoxy, more preferably methoxy;
$R_3$ is $-C(=O)-CH_3$, $-CH=N-(CH_2)_n-SO_3R_8^+$;$-CH=N-(CH_2)_n-COO^-{}_8^+$;$-CH=N-(CH_2)_n-PO_3^2-(R_8^+)2$; $-CH_2-NH-(CH_2)_n-SO_3^-R_8^+$;$-NH-(CH_2)_n-COO^-R_8^+$; or - $NH-(CH_2)_n-PO_3^{2-}(R_8^+)_2$;
$R_4$ is $-NH-(CH_2)_n-SO_3^-R_8^+$;$-NH-(CH_2)_n-COO^-R_8^+$;$-NH-(CH_2)_n-PO_3^{2-}(R_8^+)_2$; $R_8^+$ is a monovalent cation such as $K^+$, $Na^+$, $Li^+$, $NH_4^+$, more preferably $K^+$; and m is 1, and n is an integer from 1 to 10, preferably 2 or 3.

In certain embodiments of photosensitizing agents of formula II,

M is divalent Pd;
$R_1$ is $-O^-R_8^+$, $-NH-(CH_2)_n-SO_3^-R_8^+$, or $Y-R_5$ wherein Y is O, S or NH and $R_5$ is the residue of an amino acid, a peptide or a protein;
$R_2$ is $C_1$-$C_6$ alkoxy, preferably methoxy;
$R_3$ is $-C(=O)-CH_3$, $-CH= N-(CH_2)_n-SO_3^-R_8^+$; or $-CH_2-NH-(CH_2)_n-SO_3^-R_8^+$;
$R_4$ is $-NH-(CH_2)_n-SO_3^-R_8^+$; $NH-(CH_2)_n-COO^-R_8^+$; $NH-(CH_2)_n-PO_3^2-(R_8^+)_2$; $R_8^+$ is a monovalent cation, preferably $K^+$ ;
m is 1, and n is 2 or 3.

The compounds of Formula II may be synthesized according procedures described in WO 2004/045492 and US pre-grant application publication no. US 2006/01422260 A1.

In typical embodiments, the photosensitizing agent is administered intravenously. In certain embodiments, the photosensitizing agent is administered by intravenous infusion. In other embodiments, the photosensitizing agent is administered as an intravenous bolus.

In certain embodiments in which the photosensitizing agent is WST11, or pharmaceutically acceptable salt thereof, the photosensitizing agent is administered intravenously at a dose of about 2 - 6 mg/kg. In certain embodiments, the WST11 or salt thereof is administered intravenously at a dose of about 2 mg/kg, 3 mg/kg, about 4 mg/kg, about 5 mg/kg, even about 6 mg/kg. In one series of embodiments, the photosensitizing agent is Palladium $3^1$-oxo-15-methoxycarbonylmethyl-rhodobacteriochlorin $13^1$-(2-sulfoethyl) amide dipotassium salt administered intravenously at 4 mg/kg.

Light wavelength

The wavelength of light delivered will be appropriate for the chosen photosensitizing agent, and in typical embodiments will approximate an absorption maximum of the agent.

In embodiments in which the photosensitizing agent is WST11 or salt thereof, the wavelength will typically be between about 670 to about 780 nm. In various embodiments, the wavelength will be about 750 nm, including about 753 nm.

In another aspect, an improvement is provided to methods of photodynamic treatment planning. The improvement comprises setting the total length of illuminating fiber to be used for treatment based upon the planned treatment volume (PTV) and a prior-determined treatment-effective light density index threshold. As would be understood, the length of illuminating fiber refers to the length of optical fiber that is positioned in the prostate tissue and capable of delivering light to the tissue.

In typical embodiments, the total length of all illuminating fiber is calculated as the product of a prior-determined treatment-effective LDI threshold x PTV, or scalar transformation thereof. In typical embodiments, the LDI threshold and PTV are determined as above-described. In certain embodiments, the total length of all illuminating fiber is provided by a single fiber. More typically, the total length of all illuminating fiber is contributed by a plurality of fibers. In some embodiments, all fibers are of identical length. In other embodiments, the fibers differ in length.

The improvement can used in conjunction with existing methods of treatment planning that are designed to optimize the placement of optical fibers for photodynamic therapy of prostate cancer. In some embodiments, for example, the total length of all illuminating fiber calculated as above-described is used in conjunction with light diffusion-based treatment planning methods, such as that described in Davidson et al., "Treatment planning and dose analysis for interstitial photodynamic therapy of prostate cancer," Phys. Med. Biol. 54:2293-2313 (2009). In other embodiments, the total length of all illuminating fiber calculated as above-described is used in conjunction with other treatment planning algorithms.

By establishing the total length of illuminating fiber, the improvement usefully reduces the number of variables to be considered, reducing computing complexity, while ensuring that the optimized fiber placement delivers a light dose that is above the therapeutic threshold. Thus, the improvement can usefully be incorporated into software used to plan photodynamic treatment.

Thus, in another aspect, a computer program product is provided, comprising a computer usable medium having computer readable program code embodied therein, the computer readable program code adapted to be executed by a computer to implement a method for producing a patient-specific treatment plan for photodynamic therapy of prostate cancer, the method comprising a step of setting the total length of illuminating fiber to be used for treatment based upon the planned treatment volume (PTV) and a prior-determined treatment-effective light density index threshold. In typical embodiments, the PTV and prior-determined treatment-effective light density index threshold are calculated as above-described, and the total length of illuminating fiber is calculated as the product of a prior-determined treatment-effective LDI threshold x PTV, or scalar transformation thereof.

Further advantages and features are shown in the following Example, which is presented by way of illustration and is not to be construed as limiting the scope of the present invention.

## 4. EXAMPLES

### Example 1: Light density index predicts early efficacy of focal vascular targeted photodynamic treatment of prostate cancer

Materials and Methods

Palladium $3^1$-oxo-15-methoxycarbonylmethyl-rhodobacteriochlorin $13^1$-(2-sulfoethyl) amide dipotassium salt was prepared as described in WO 2004/045492 and US 2006/0142260, the disclosures of which are incorporated herein by reference in their entireties.

Men with low risk organ-confined prostate cancer (Gleason 3 +3 on minimum 10 core TRUS biopsy) were recruited into two consecutive studies: PCM201 (a dose escalation study) or PCM203 (a confirmatory study). The vascular-targeted photodynamic therapy ("VTP") procedure, carried out under general anesthesia, involved administration of TOOKAD® Soluble intravenously at 4 mg/kg, which was then activated by low power laser light delivered locally to the prostate via a brachytherapy-style transperineal template with a light density of 200 J/cm. Planned treatment volume ("PTV", in mls) was determined by the location of tumor on biopsy and by MRI, and varied in volume from less than one lobe, to the whole prostate.

The Light Density Index ("LDI") was calculated as LDI = $\sum(n)L/PTV$, where $\sum(n)L$ is the total length of all illuminating fibers (in cm), and PTV is the planned treatment volume, in mls.

Early treatment effect was determined as the proportion of the PTV which showed lack of uptake of gadolinium

on 1 week MRI. This was also correlated with the result of 6 month transrectal ultrasound (TRUS)-guided biopsy (positive or negative for any cancer).

The correlation between LDI and the volume of tissue necrosis at day 7 and with the rate of negative biopsies at Month 6 was assessed in search of a threshold.

Results

90 men were treated with a TOOKAD® Soluble dose of 4mg/kg and a light dose of 200J/cm in the two studies. Of these 89 were analyzable for LDI. Results using an LDI threshold of 1 are presented below.

| | **Treatment effect on 1 week MRI as % of PTV (n lobes)** | | **% of negative biopsies (6 months) (n lobes))** | |
|---|---|---|---|---|
| | PCM201 | PCM203 | PCM201 | PCM203 |
| **LDI <1** | 59 (17) | 60 (22) | 31 (4/17) | N/A |
| **LDI≥ 1** | 95 (12) | 94 (28) | 83 (10/12) | N/A |
| **P** | <0.01 | <0.01 | <0.01 | - |
| (N/A - not yet available) | | | | |

Conclusion

LDI is a reliable predictor of treatment effect using TOOKAD® Soluble VTP, both in terms of effect seen on 1 week MRI, and 6 month biopsy.

While various specific embodiments have been illustrated and described, it will be appreciated that various changes can be made without departing from the scope of the invention(s).

## Claims

1. A computer program product comprising a computer usable medium having computer readable program code embodied therein, the computer readable program code adapted to be executed by a computer to implement a method for producing a patient-specific treatment plan for photodynamic therapy of prostate cancer, the method comprising:

   setting the total length of illuminating fibers needed for effective therapy based upon the planned treatment volume (PTV) and a prior-determined treatment-effective light density index, LDI, threshold , wherein the treatment-effective LDI threshold is prior-determined from historical data from use of the same photosensitizing agent, and
   wherein the total length of illuminating fibers is calculated as the product of PTV and said prior-determined treatment-effective light density index threshold, or scalar multiple thereof.

2. The computer program product of claim 1, wherein the treatment-effective LDI threshold is prior-determined from historical data from use of the same photosensitizing agent, administered at the same systemic dosage.

3. The computer program product of claim 2, wherein the treatment-effective LDI threshold is prior-determined from historical data from use of the same photosensitizing agent, administered at the same systemic dosage, and same wavelength of delivered light.

4. The computer program product of claim 3, wherein the treatment-effective LDI threshold is prior-determined from historical data from use of the same photosensitizing agent, administered at the same systemic dosage, same irradiating light wavelength, and same light density.

5. An assistance method, implemented by computer, for the planning of treatment of a patient by photodynamic therapy, in which a predefined photosensitizing agent must be administered to the patient, and then subjected to illumination at a predetermined wavelength through at least one illuminating fiber designed to be introduced over a length of insertion into the treatment area, **characterized in that** it includes the following steps:

calculating the planned treatment volume, PTV, of the treatment area;
determining a treatment-effective light density index, LDI, threshold, wherein the treatment-effective LDI threshold is prior-determined from historical data from use of the same photosensitizing agent; and then
setting the total length of said at least one illuminating fiber to be used for treatment based upon the planned treatment volume, PTV, and said prior-determined treatment-effective light density index threshold, wherein the total length of illuminating fiber is calculated as the product of PTV and said prior-determined treatment-effective light density index threshold or scalar multiple thereof.

**6.** The assistance method of claim 5, wherein the treatment-effective LDI threshold is prior-determined from historical data from use of the same photosensitizing agent, administered at the same systemic dosage.

**7.** The assistance method of claim 6, wherein the treatment-effective LDI threshold is prior-determined from historical data from use of the same photosensitizing agent, administered at the same systemic dosage, and same wavelength of delivered light.

**8.** The assistance method of claim 7, wherein the treatment-effective LDI threshold is prior-determined from historical data from use of the same photosensitizing agent, administered at the same systemic dosage, same irradiating light wavelength, and same light density.

**Patentansprüche**

**1.** Computerprogrammprodukt, umfassend ein mit einem Computer verwendbares Medium, das computerlesbarem Programmcode darauf enthalten aufweist, wobei der computerlesbare Programmcode so ausgelegt ist, dass er von einem Computer ausgeführt wird, um ein Verfahren zur Erzeugung eines patientenspezifischen Behandlungsplans für photodynamische Prostatakrebstherapie zu implementieren, wobei das Verfahren umfasst:

Einstellen der Gesamtlänge von Beleuchtungsfasern, die zur wirksamen Therapie basierend auf dem geplanten Therapievolumen (PTV) benötigt werden, und einer vorherbestimmten behandlungswirksamen Lichtdichteindex, LDI,-Schwelle, wobei die behandlungswirksame LDI-Schwelle aus historischen Daten von der Verwendung des gleichen Photosensibilisierungsmittels vorherbestimmt ist, und
wobei die Gesamtlänge der Beleuchtungsfasern als das Produkt des PTVs und der vorherbestimmten behandlungswirksamen Lichtdichteindexschwelle oder ein skalares Vielfaches davon berechnet wird.

**2.** Computerprogrammprodukt nach Anspruch 1, wobei die behandlungswirksame LDI-Schwelle aus historischen Daten von der Verwendung des gleichen Photosensibilisierungsmittels, verabreicht mit der gleichen systemischen Dosierung, vorherbestimmt ist.

**3.** Computerprogrammprodukt nach Anspruch 2, wobei die behandlungswirksame LDI-Schwelle aus historischen Daten von der Verwendung des gleichen Photosensibilisierungsmittels, verabreicht mit der gleichen systemischen Dosierung und der gleichen Wellenlänge des zugeführten Lichts, vorherbestimmt ist.

**4.** Computerprogrammprodukt nach Anspruch 3, wobei die behandlungswirksame LDI-Schwelle aus historischen Daten von der Verwendung des gleichen Photosensibilisierungsmittels, verabreicht mit der gleichen systemischen Dosierung, der gleichen Bestrahlungslichtwellenlänge und der gleichen Lichtdichte, vorherbestimmt ist.

**5.** Computerimplementiertes Verfahren zur Unterstützung der Planung der Behandlung eines Patienten durch photodynamische Therapie, wobei ein vordefiniertes Photosensibilisierungsmittel an den Patienten verabreicht und anschließend Beleuchtung bei einer vorbestimmten Wellenlänge durch mindestens eine Beleuchtungsfaser unterzogen werden muss, die so ausgelegt ist, dass sie über eine Einführungslänge in den Behandlungsbereich eingeführt wird, **dadurch gekennzeichnet, dass** es die folgenden Schritte umfasst:

Berechnen des geplanten Behandlungsvolumens, PTVs, des Behandlungsbereichs;
Bestimmen einer behandlungswirksamen Lichtdichteindex, LDI,- Schwelle, wobei die behandlungswirksame LDI-Schwelle aus historischen Daten von der Verwendung des gleichen Photosensibilisierungsmittels vorherbestimmt wird; und anschließendes
Einstellen der Gesamtlänge der mindestens einen Beleuchtungsfaser, die zur Behandlung verwendet werden soll, basierend auf dem geplanten Behandlungsvolumen, PTV, und der vorherbestimmten behandlungswirksamen Lichtdichteindexschwelle, wobei die Gesamtlänge der Beleuchtungsfaser als das Produkt des PTVs und der vorherbestimmten behandlungswirksamen Lichtdichteindexschwelle oder ein skalares Vielfaches davon berechnet wird.

**6.** Unterstützungsverfahren nach Anspruch 5, wobei die behandlungswirksame LDI-Schwelle aus historischen Daten von der Verwendung des gleichen Photosensibilisierungsmittels, verabreicht mit der gleichen systemischen Dosierung, vorherbestimmt wird.

**7.** Unterstützungsverfahren nach Anspruch 6, wobei die behandlungswirksame LDI-Schwelle aus historischen Daten von der Verwendung des gleichen Photosensibilisierungsmittels, verabreicht mit der gleichen systemischen Dosierung und der gleichen Wellenlänge des zugeführten Lichts, vorherbestimmt wird.

**8.** Unterstützungsverfahren nach Anspruch 7, wobei die behandlungswirksame LDI-Schwelle aus historischen Daten von der Verwendung des gleichen Photosensibilisierungsmittels, verabreicht mit der gleichen systemischen Dosierung, der gleichen Bestrahlungslichtwellenlänge und der gleichen Lichtdichte, vorherbestimmt wird.

**Revendications**

**1.** Produit-programme d'ordinateur comprenant un support utilisable par un ordinateur dans lequel un code de programme pouvant être lu par un ordinateur est mis en oeuvre, le code de programme pouvant être lu par un ordinateur étant conçu pour être exécuté par un ordinateur pour effectuer un procédé pour produire un plan de traitement spécifique à un patient pour une thérapie photodynamique du cancer de la prostate, le procédé comprenant :

l'établissement de la longueur totale des fibres de rayonnement nécessaire pour une thérapie efficace sur la base du volume de traitement planifié (PTV) et d'un seuil d'indice de densité de lumière, LDI, efficace pour un traitement déterminé au préalable, dans lequel le seuil de LDI efficace pour un traitement est déterminé au préalable à partir de données d'historique provenant de l'utilisation du même agent de photosensibilisation, et dans lequel la longueur totale des fibres de rayonnement est calculée en tant que produit du PTV et dudit seuil d'indice de densité de lumière efficace pour un traitement déterminé au préalable, ou d'un multiple scalaire de celui-ci.

**2.** Produit-programme d'ordinateur selon la revendication 1, dans lequel le seuil de LDI efficace pour un traitement est déterminé au préalable à partir de données d'historique provenant de l'utilisation du même agent de photosensibilisation, administré au même dosage systémique.

**3.** Produit-programme d'ordinateur selon la revendication 2, dans lequel le seuil de LDI efficace pour un traitement est déterminé au préalable à partir de données d'historique provenant de l'utilisation du même agent de photosensibilisation, administré au même dosage systémique, et à la même longueur d'onde de la lumière délivrée.

**4.** Produit-programme d'ordinateur selon la revendication 3, dans lequel le seuil de LDI efficace pour un traitement est déterminé au préalable à partir de données d'historique provenant de l'utilisation du même agent de photosensibilisation, administré au même dosage systémique, à la même longueur d'onde de lumière de rayonnement, et à la même densité de lumière.

**5.** Procédé d'assistance, effectué par un ordinateur, pour la planification du traitement d'un patient par une thérapie photodynamique, dans lequel un agent de photosensibilisation prédéfini doit être administré au patient, et ensuite soumis à un rayonnement à une longueur d'onde prédéterminée à travers au moins une fibre de rayonnement conçue pour être introduite sur une longueur d'insertion dans la zone de traitement, **caractérisé en ce qu'**il comprend les étapes suivantes :

de calcul du volume de traitement planifié, PTV, de la zone de traitement ;
de détermination d'un seuil d'indice de densité de lumière, LDI, efficace pour un traitement, dans lequel le seuil de LDI efficace pour un traitement est déterminé au préalable à partir de données d'historique provenant de l'utilisation du même agent de photosensibilisation ; et ensuite
d'établissement de la longueur totale de ladite au moins une fibre de rayonnement à utiliser pour un traitement sur la base du volume de traitement planifié, PTV, et dudit seuil d'indice de densité de lumière efficace pour un traitement déterminé au préalable, dans lequel la longueur totale de la fibre de rayonnement est calculée en

tant que produit du PTV et dudit seuil d'indice de densité de lumière efficace pour un traitement déterminé au préalable, ou d'un multiple scalaire de celui-ci.

**6.** Procédé d'assistance selon la revendication 5, dans lequel le seuil de LDI efficace pour un traitement est déterminé au préalable à partir de données d'historique provenant de l'utilisation du même agent de photosensibilisation, administré au même dosage systémique.

**7.** Procédé d'assistance selon la revendication 6, dans lequel le seuil de LDI efficace pour un traitement est déterminé au préalable à partir de données d'historique provenant de l'utilisation du même agent de photosensibilisation, administré au même dosage systémique, et à la même longueur d'onde de la lumière délivrée.

**8.** Procédé d'assistance selon la revendication 7, dans lequel le seuil de LDI efficace pour un traitement est déterminé au préalable à partir de données d'historique provenant de l'utilisation du même agent de photosensibilisation, administré au même dosage systémique, à la même longueur d'onde de lumière de rayonnement, et à la même densité de lumière.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004045492 A
- US 200601422260 A1
- US 20060142260 A


### Non-patent literature cited in the description


- **KOUDINOVA et al.** Photodynamic therapy with Pd-Bacteriopheophorbide (TOOKAD): successful in vivo treatment of human prostatic small cell carcinoma xenografts. *Int. J. Cancer,* 2003, vol. 104 (6), 782-9
- **WEERSINK et al.** Techniques for delivery and monitoring of TOOKAD (WST09)-mediated photodynamic therapy of the prostate: clinical experience and practicalities. *J Photochem Photobiol B.,* 2005, vol. 79 (3), 211-22
- **TRACHTENBERG et al.** Vascular targeted photodynamic therapy with palladium-bacteriopheophorbide photosensitizer for recurrent prostate cancer following definitive radiation therapy: assessment of safety and treatment response. *J Urol.,* 2007, vol. 178 (5), 1974-9
- **TRACHTENBERG et al.** Vascular-targeted photodynamic therapy (padoporfin, WST09) for recurrent prostate cancer after failure of external beam radiotherapy: a study of escalating light doses. *BJU Int.,* 2008, vol. 102 (5), 556-62
- **MADAR-BALAKIRSKI et al.** Permanent occlusion of feeding arteries and draining veins in solid mouse tumors by vascular targeted photodynamic therapy (VTP) with Tookad. *PLoS Once,* 2010, vol. 5 (4), e10282
- **MAZOR et al.** WST-11, A Novel Water-soluble Bacteriochlorophyll Derivative: Cellular Uptake, Pharmacokinetics, Biodistribution and Vascular-targeted Photodynamic Activity Using melanoma Tumors as a Model. *Photochemistry & Photobiology,* 2005, vol. 81, 342-351
- **BRANDIS et al.** Novel Water-soluble Bacteriochlorophyll Derivatives for Vascular-targeted Photodynamic Therapy: Synthesis, solubility, Phototoxicity and the Effect of Serum Proteins. *Photochemistry & Photobiology,* 2005, vol. 81, 983-993
- **ASHUR et al.** Photocatalytic Generation of Oxygen Radicals by the Water-Soluble Bacteriochlorophyll Derivative WST-11, Noncovalently Bound to Serum Albumin. *J. Phys. Chem. A,* 2009, vol. 113, 8027-8037
- **DAVIDSON et al.** Treatment planning and dose analysis for interstitial photodynamic therapy of prostate cancer. *Phys. Med. Biol.,* 2009, vol. 54, 2293-2313
- **DAVIDSON et al.** Treatment planning and dose analysis for interstitial photodynamic therapy of prostate cancer. *Phys. Med. Biol,* 2009, vol. 54, 2293-2313